# EUROPEAN PATENT APPLICATION

(11) **EP 3 711 805 A1**
(43) Date of publication of application: **23.09.2020**
(21) Application number: 20162805.4
(22) Date of filing: 12.03.2020
(51) Int. Cl.: A61M 16/04, A61M 16/00

(54) **MEDICAL DEVICE TUBE COMPRISING A PRESSURE SENSOR**

(30) Priority: 19.03.2019 EP 19163847
(71) Applicant: CSEM Centre Suisse d'Electronique et de Microtechnique SA - Recherche et Développement, 2002 Neuchâtel (CH); Universitäts-kinderspital Beider Basel, 4056 Basel (CH)
(72) Inventor: ERB, Thomas, 4107 Ettingen (CH); GLASER, Nicolas, 4056 Basel (CH); ZANELLA, Frédéric, 68300 Saint-Louis (FR)
(74) Representative: Bovard SA Neuchâtel

(57) **Abstract**

The invention relates to a medical device (1) for trachea pressure measurement comprising a tracheal tube defining an inner surface (12a) and an outer surface (12b) and having a proximal end (1a) and a distal end (1b), configured to be inserted at least partially into the trachea of a subject or animal to deliver respiratory gases to the subject.

The medical device comprises at least one capacitive pressure sensor (20) arranged at said distal end (1b).

## Description

### Technical Field

The invention relates generally to the field of medical device, and more particularly to airway devices comprising tracheal tubes connected to pressure measurement systems. The invention relates more precisely to tracheal tubes connected to a pressure sensor device.

The invention also relates to a method of fabrication of a pressure sensor and its integration in a tracheal tube.

### Background of the art

In the course of treating a mammal such as a human or an animal, a tube or other medical device may be used to control the flow of air, food, fluids or other substances into the mammal, typically a patient. For example, tracheal tubes may be used to control the flow of air or other gases through a patient's or animal's trachea and into the lungs, for example during the patient ventilation. Such tracheal tubes may have different natures, such as endotracheal tubes, tracheotomy tubes, or transtracheal tubes.

Medical intubation tubes require to measure the air pressure in such tubes. In particular a clinical person desires to measure and monitor the air pressure at the lower part of such tubes, during operations such as surgery and intensive care. The air pressure is preferably known at the tip of such tubes i.e. to the side of the lower part of the trachea. The reason for this is that it has been demonstrated that the current respiration pressure monitoring approach is not very accurate nor stable over time.

Most current approaches consist in measuring the pressure at the respiratory side and estimating the air pressure at the tip of the medical tubing by using a mechanical model of all the tubing parts located between a machine located outside the patient and the air outlet including the intubation tube.

Document US 2011/144514 A1 illustrates such a system wherein the gas pressure at the distal end of the tubing is measured by using an additional tubing that is connected to an outside pressure transducer that is always situated outside the body of the subject.

Document US 2003/015202 describes another system, presenting the same problems and limitations as in US 2012/144514 A1, and wherein the medical tubing comprises a pressure line connected to a pressure monitoring subsystem.

Document WO 2012/015587 A2 describes another medical device tube having a suction lumen and an associated suctioning system and wherein a first and a second pressure sensor are placed at two different locations situated at the site opposite to the end of the lumen that is inserted in the body. The device described in WO 2012/015587 A2 presents the same limitations as the ones of the device described in US 2012/144514 A1. In addition to these limitations, these sensors measure only the pressure on/in the suction lumen (different from the respiratory tube) and are located on the proximal end side of the inflated cuff (not between the tube distal end and the cuff). This to detect the presence of liquid secretions within the suction lumen or on it. So these sensors cannot measure in any way the tracheal air pressure.
Some sensing principles have been disclosed to apply pressure sensors inside a medical tubing such as pressure sensors or pressure measurement systems to measure and monitor the pressure of cuffs integrated into the medical tubing. Integrated pressure sensors are only intended to measure and monitor the pressure of inflated cuffs exerted against the tracheal tube walls.
Document WO 01/30431 describes a medical tubus comprising two pressure sensors for detecting dynamically the endotracheal pressure. This system is quite complex as the two sensors have to be integrated into the wall of the medical tubus and connected by a channel provided inside said wall which provides an expensive arrangement. The system described in WO 01/30431 presents also a risk of wrong pressure monitoring if the channel inside said wall gets compressed, for example in the case of a flexible medical tubing. The system described in WO 01/30431 has another disadvantage linked to the fact that quite thick and so nonflexible walls have to be used to allow providing a channel inside the walls.

Another important aspect of medical tubing is that they are disposable and thus must have a low cost. Existing approaches to integrate pressure sensors into medical tubing have failed because of their high cost and/or their low reliability.

There is thus a need to provide a medical tubing that incorporates a pressure measurement system into the distal end of a medical tubing so that in-situ measurements of the pressure of air and gases can be measured reliably and at low cost by a medical machine during surgery and intensive care of a patient.

### Summary of the invention

It is an objective of the invention to provide an improved medical intubation tube that comprises a pressure sensor so that the gas pressure at a distal end of an intubation tube can be measured and monitored in a reliable and cheap way.

The medical intubation tube of the invention comprises a gas pressure sensor integrated at a distal end, i.e. the end that is intended to be inserted in the lower part of the trachea of a mammal, such as a patient. Adding a low-cost pressure sensor to at least its distal, i.e. lower end of a disposable intubation tube provides a distinct differentiator on the market of medical devices.

The medical device of the invention is mainly intended for trachea pressure measurement and comprises, a tracheal tube defining an inner surface 12a and an outer surface and having a proximal end and a distal end, configured to be inserted at least partially into the trachea of a subject or animal to deliver respiratory gases to the subject

More precisely at least one capacitive pressure sensor is arranged at said distal end of the tracheal tube .

In an embodiment said tracheal tube comprises signal transmission means arranged to transmit signals provided by said pressure sensor to a signal conversion device situated to the side of said tube opposite to said distal end.

In an embodiment said signals are electrical signals and wherein said signal transmission means comprise at least one coaxial cable. In an embodiment said signals are optical signals and wherein said signal transmission means comprise at least one optical waveguide.

In an embodiment said pressure sensor is arranged into a closed deformable cavity (sealed by 40 and 42). In an embodiment said pressure sensor comprises at least two thin deformable layers each said deformable layers comprising at least one electrical conducting electrode

In an embodiment said at least one pressure sensor is arranged to said outer surface . In a variant said at least one pressure sensor is arranged to said inner surface. In another advantageous embodiment at least one pressure sensor is arranged to said outer surface and at least one pressure sensor is arranged to said inner surface .

In an embodiment said cavity is filled at least partially with a viscoelastic and/or an elastomeric material.

In an embodiment said cavity comprises a plurality of deformable pillars configured to separate said two thin layers. In a variant said two thin layers are separated by a gap being less than 1 mm defined over the whole plane of said gap, preferably less than 200 microns.

In an embodiment said pillars have a density, defined in the central plane of said pressure sensor, of more than 10, preferable more than 50, even more preferably more than 100 pillars per square mm. In a variant different types of deformable pillars are arranged between said at least two thin layers.

In an embodiment said cavity comprises a layer of porous deformable material between said two thin layers.

In an embodiment the medical device comprises a cuff and wherein at least one pressure sensor is arranged to the side of said cuff opposite to said proximal end of the tube.

In an embodiment said pressure sensor is arranged into a sealed cavity filled with a gas at a predetermined pressure.

In an embodiment the at least one pressure sensor is arranged to said outside surface, and comprises at least one protection layer that reduces considerably friction with body tissue or/and which is biocompatible.

### Brief description of the drawings

Further details of the invention will appear more clearly upon reading the following description in reference to the appended figures:
- Figure 1 illustrates a view of the medical device of the invention;
- Figures 2-7 illustrate cross sections of the medical tube of the invention at the location where the pressure sensor is fixed on the medical tube;
- Figure 8 illustrates a detailed cross section of the pressure sensor of the medical device of the invention illustrating a portion of the medical tube on which it is fixed;
- Figure 9 illustrates a portion of the medical device of the invention, comprising an inflated cuff.
- Figure 10 illustrates the response of the working prototype compared to a reference pressure sensor when the air pressure is modulated by a medical ventilator.

### Detailed description and embodiments of the invention

The present invention will be described with respect to particular embodiments and with reference to certain drawings but the invention is not limited thereto. The drawings described are only schematic and are non-limiting. In the drawings, the size of some of the elements may be exaggerated and not drawn on scale for illustrative purposes. The dimensions and the relative dimensions do not correspond to actual reductions to the practice of the invention.

It is to be noticed that the term "comprising" in the description and the claims should not be interpreted as being restricted to the means listed thereafter, i.e. it does not exclude other elements.

Reference throughout the specification to "an embodiment" means that a particular feature, structure or characteristic described in relation with the embodiment is included in at least one embodiment of the invention. Thus appearances of the wording "in an embodiment" or, "in a variant", in various places throughout the description, are not necessarily all referring to the same embodiment, but several. Furthermore, the particular features, structures or characteristics may be combined in any suitable manner, as would be apparent to a skilled person from this disclosure, in one or more embodiments. Similarly, various features of the invention are sometimes grouped together in a single embodiment, figure or description, for the purpose of making the disclosure easier to read and improving the understanding of one or more of the various inventive aspects. Furthermore, while some embodiments described hereafter include some but not other features included in other embodiments, combinations of features if different embodiments are meant to be within the scope of the invention, and from different embodiments. For example, any of the claimed embodiments can be used in any combination. It is also understood that the invention may be practiced without some of the numerous specific details set forth. In other instances, not all structures are shown in detail in order not to obscure an understanding of the description and/or the figures.

The term "gas" includes here all types of gases that are usually used in medical interventions, and is typically air but not exclusively air.

The term "distal" means here to the side of the device that is intended to be inserted in a body. The wording "subject" or "patient" is used broadly here and may also comprise any type of animals, such as mammals. The device of the invention is mainly used for the insertion in trachea but may also be modified to be used in other parts of a living body such as the ear, the stomach or any other place of a body wherein a local gas pressure has to be monitored. It is also understood that the term "tubing" is defined largely as any device that is arranged to transport a gas, independent of its shape or material composition, i.e. the tubing must not be necessarily be a flexible plastic tubing. The wording "medical tubing" relates to tubings that may be inserted in bodies, preferably living bodies. Some applications may address the problem of measuring pressure at a distance in a medical system, such as a medical calibration system.

Tubings 10 of the invention have an inner surface 12a and an outer surface 12b.

The invention includes the following embodiments.

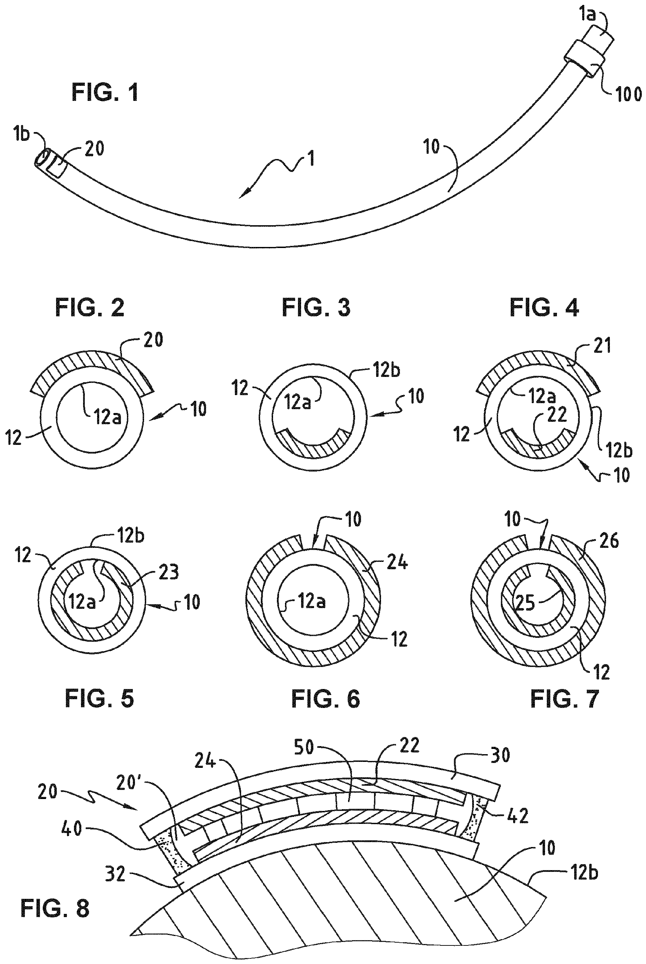

Fig. 1 illustrates a medical device 1 comprising a tubing 10 of the invention that comprises a pressure sensor 20 integrated to its distal end 1b, defined also as the lower end, meaning the end to the side of the lower part of the trachea or otherwise said to the side away from a machine to which the medical tubing may be connected. The distal end 1b is alternatively defined as the end of the medical tubing 1 that is intended to be inserted in a part of a mammal or human, such as the lungs or its proximity. The pressure sensor 20 is preferably a capacitive sensor, but may be another type of pressure sensor.

It has to be understood that the medical device 1 and the medical tubing 10 of the invention is not limited to measure the pressure of air or gases directed to or provided by the lungs, but may also be configured to be inserted in other parts of the body wherein the measurement of gas pressure may be needed. For example a miniaturized embodiment of the invention may be configured to measure the air pressure inside the volume of the internal ear.
- Figures 2-7 illustrate cross sections of embodiments of the sensor 20 that is integrated into or on the distal end 1b of the medical tubing of the invention:
- Fig.2 and Fig.6 illustrate embodiments of a sensor 20 that has the form of an arc arranged on the outside surface 12b of the medical tubing 10;
- Fig.3 and Fig. 5 illustrate embodiments of a sensor 20 that has the form of an arc arranged on the inside surface 12a of the medical tubing 10;
- Fig.4 and Fig.7 illustrate embodiments of a distal end of the tubing comprising a first sensor 21 that has the form of an arc arranged on the outside surface 12b of the medical tubing 10, and a second sensor 22 that has the form of an arc arranged on the inside surface 12a of the medical tubing 10.

In embodiments more than one capacitive pressure sensor 20 may be arranged at said distal end 1b of the medical tube 10. Capacitive sensors 20 may be arranged on top of each other or in proximity or may be partially overlapping. In variants a distal end 1b may comprise different types of pressure sensors and at least one of them may be a capacitive sensor 20.

In an embodiment said tracheal tube 1 comprises signal transmission means arranged to transmit signals provided by said pressure sensor 20 to a signal conversion device situated to the side of said tube 10, opposite to said distal end 1b

In an advantageous arrangement said signals are electrical signals and wherein said signal transmission means 11 comprise at least one coaxial cable. The signal transmission cable may also be a flat cable and may be integrated in the medical tube 10. It is also understood that a plurality of transmission wires or cables may be integrated on or inside the medical tube and at least a part of said transmission wires may have outputs arranged at different parts of said tube 10.

In an embodiment said signals are optical signals and wherein said signal transmission means 11 comprise at least one optical waveguide.

In an embodiment said pressure sensor 20 is arranged into a closed deformable cavity 20'. The term "closed" has to be understood broadly and may be tight or sealed. The volume of the deformable cavity may have different shapes, for example the shape of a gap between two surfaces

In an embodiment said pressure sensor 20 comprises at least two thin deformable layers 30, 32, each said deformable layers 30, 32 comprising at least one electrical conducting electrode 22, 24. Said at least two thin deformable layers 30, 32 are preferably made of a polymer less than 1 mm thick but not necessarily so. Said electrical conducting electrodes 22, 24 are preferably less than 50 µm thick but not necessarily so.

In an embodiment said cavity 20' is filled at least partially with a viscoelastic and/or an elastomeric material.

In an advantageously embodiments, illustrated in Fig.8, said cavity 20' comprises at least a plurality of microstructured deformable pillars 50 configured to separate said two thin deformable layers 30, 32 .

In an embodiment said two thin layers 30, 32 are separated by a gap being less than 1 mm defined over the whole plane of said gap, this gap being preferably thinner than (less than) 200 microns.

In an embodiment said pillars 50 have a density, defined in the central plane of said pressure sensor 20, of more than 10, preferable more than 50, even more preferably more than 100 pillars 50 per square mm.

In an embodiment different types of deformable pillars 50 are arranged between said at least two thin layers.

In variants said pillars may have the shape of bars or cylinders or hollow cylinders or domes or pyramids.

In an embodiment said cavity 20' comprises a layer of porous deformable material between said two thin layers 30, 32. Said porous deformable material may be a sponge-like material having a random distribution of internal void space.

Ideally the sensor is sealed as illustrated in the embodiment of Fig.8. Said sealing 40, 42 has to be understood broadly as there exist a wide variety of techniques and/or arrangements to avoid that the inside of the pressure sensor 20' becomes in contact with the ambient gas and body fluids. Sealing may be assured by glues and/or materials that repell fluids. The obtained capacitive pressure sensor 20 should be above 1 pF at ambient pressure.

In an embodiment the medical device 1 comprises a cuff and wherein at least one pressure sensor 20 is arranged to one side of said cuff. The pressure sensor is arranged preferably near to said distal end

In an embodiment said pressure sensor 20 is arranged into a sealed cavity 20' filled with a gas at a predetermined pressure.

In an embodiment said pressure sensor 20 comprises additional electrode layers used for electrically shielding the capacitive sensor for instance between one of said thin deformable layer 32 and said outer surface 12b, and/or on top of one of said thin deformable layer 30, and/or on said sealing 40, 42. In an embodiment the at least one pressure sensor 20 is arranged to said outside surface 12b, and comprises at least one protection layer that reduces considerably friction with body tissue and/or is biocompatible.

In embodiments the pressure sensor is a printed pressure sensor. In an advantageous embodiment the pressure sensor 20 and electrical signal transmission means 11 (including coaxial cables) are realized by additive manufacturing techniques. In another embodiment, a part of the pressure sensor is made by vacuum coating techniques and another part is made by additive manufacturing techniques.

It is also understood that a wide variety of substrates may be used as support of the pressure sensor 20, and also that a wide variety of protection and intermediate layers or structures may be used to adapt the pressure sensor on said medical tube. For instance the distal end of the medical tube may comprise aperture and the pressure sensor may comprise protuberances so that the pressure sensor can be clipped on said distal end. In a variant the pressure sensor 20 may be glued to said medical tube or overmoulded.

It is further understood that the portion of the medical tube 10 that is in proximity of said distal end may be configured so that the pressure sensor may be arranged at least partially in the wall 12 of the medical tube 10.

In a variant the sensor bottom electrode 22 is directly patterned on the said outer surface 12b.

In variants the medical tube 10 may comprise at least another tube and may comprise a plurality of pressure sensors on said at least another tube.

In embodiments at least two pressure sensors may be arranged to be in fluidic and/or optical and/ or electrical communication. In variants said pressure sensors may comprise other type of sensors such as temperature sensors, flow sensors, CO2 sensor or whatever sensor that might be needed to measure or control local physical, optical, biological or chemical conditions in proximity of said distal end.

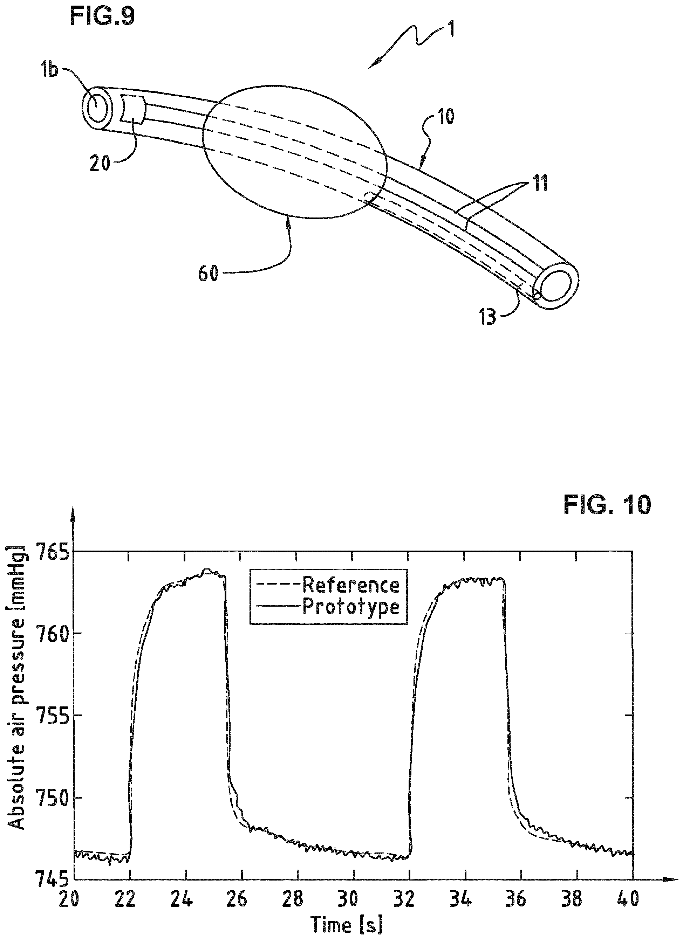

It is generally understood that the invention is not limited to a medical device 1 that comprises only a medical tube. Advantageously the medical device may comprise connection means such as a ring 100 arranged on said medical tube 10 so that the medical tube 10 can be connected to a pressure measurement system or to a medical ventilator. The medical device 1 may also comprise a cuff 60 and its dedicated inflation tube 13 such as illustrated in Fig.9 . Also, as illustrated in Fig.9 the medical device 1 may comprise more than 1 tube 10 and more than 1 cuff 60.

### Experimental results

Fig. 10 illustrates the response of the working prototype compared to a reference pressure sensor (Bosch BME280) when the air pressure is modulated by a medical ventilator. The averaged acquisition rate of both Reference and Prototype sensors here was 38.5 Hz.

The prototype measured in Fig. 10 was realized by screen-printing a conductive ink (less than 10 µm of silver) on two flexible plastic films (such as polyamide). In Fig. 8 the corresponding structures are referred to as 22, 24, 30 and 32, respectively. A dielectric elastomer (such as PDMS) was deposited on the bottom flexible plastic film 32 and micropillars 50 were structured within this elastomeric layer, using a thermal-NIL process. The two flexible films 32 and 30 were assembled together and sealed with an adhesive (such as silicone-based elastomeric adhesive) 40, 42 in order to close the cavity 20' in ambient atmosphere. The resulting sensor 20 is around 0.2 mm thick and its footprint is around 200 mm². The prototype sensor 20 was glued to a tube outer surface following the variant shown in Fig. 2. At ambient pressure its capacitance is about 28pF.
For the measurement depicted in Fig. 10 an artificial lung model was used together with a medical ventilator and with a plastic tube modelling the trachea. An endotracheal tube equipped with the prototype sensor was inserted into the trachea model together with a reference sensor (Bosch BME280). This reference sensor, because of its dimensions cannot be well-integrated to the tube and is protruding, it is more costly than the sensor subject of the invention and is more difficult to connect and assemble to intubation tubes.

The following table 1 summaries the range of some measured performance parameters.

| Characteristic | Measured performance |
|---|---|
| Dynamic range | From -50 mmHg to 100 mmHg relative to ambient atmospheric pressure |
| Acquisition rate | 21-61 Hz |
| Sensitivity | 15-30 fF/mmHg |
| Resolution | 0.4-0.9 mmHg |

## Claims

1. A medical device (1) for trachea pressure measurement comprising a tracheal tube (10) defining an inner surface (12a) and an outer surface (12b) and having a proximal end (1a) and a distal end (1b), configured to be inserted at least partially into the trachea of a subject or animal to deliver respiratory gases to the subject
**characterized in that.**
- at least one capacitive pressure sensor (20) is arranged at said distal end (1b)
- said tracheal tube (10) comprising signal transmission means arranged to transmit signals provided by said pressure sensor (20) to a signal conversion device situated to the side of said tube (10), opposite to said distal end (1b).

2. The medical device according to claim 1 wherein said signals are electrical signals and wherein said signal transmission means (11) comprise at least one coaxial cable.

3. The medical device (1) according to claim 1 or claim 2 wherein said signals are optical signals and wherein said signal transmission means (11) comprise at least one optical waveguide.

4. The medical device according to any one of claims 1 to claim 4 wherein said pressure sensor (20) is arranged into a closed deformable cavity (20').

5. The medical device according to any one of claims 1 to 5 wherein said pressure sensor (20) comprises at least two thin deformable layers (30, 32), each said thin deformable layers (30, 32) comprising at least one electrical conducting electrode (22).

6. The medical device according to any one of claims 1 to 6 wherein said at least one pressure sensor (20) is arranged to said inner surface (12a).

7. The medical device according to any one of claims 1 to 6 wherein said at least one pressure sensor (20) is arranged to said outside surface (12b).

8. The medical device according to any one of claims 7 or claim 8 wherein at least one pressure sensor (20) is arranged to said outer surface (12b) and wherein at least one pressure sensor (20) is arranged to said inner surface (12a).

9. The medical device (1) according to any one of claims 4 to 9 wherein said cavity (20') is filled at least partially with a viscoelastic and/or an elastomeric material.

10. The medical device (1) according to any one of claims 4 to 10 wherein said cavity (20') comprises a plurality of deformable pillars (50) configured to separate said two thin deformable layers (30, 32) .

11. The medical device (1) according to any one of claims 5 to 10 wherein said two thin deformable layers (30, 32) are separated by a gap being less than 1 mm defined over the whole plane of said gap, preferably less than 200 microns.

12. The medical device (1) according to claim 10 or claim 11 wherein said pillars (50) have a density, defined in the central plane of said pressure sensor (20), of more than 10, preferable more than 50, even more preferably more than 100 pillars (50) per square mm.

13. The medical device (1) according to any one of claims 10 to claim 12 wherein different types of deformable pillars (50) are arranged between said at least two thin deformable layers.

14. The medical device (1) according to any one of claims 4 to 10 wherein said cavity (20') comprises a layer of porous deformable material between said two thin deformable layers (30, 32) .

15. The medical device (1) according to any one of claims 1 to 14 and comprises a cuff and wherein at least one pressure sensor (20) is arranged to the side of said cuff opposite to said proximal end of said tube.

16. The medical device (1) according to any one of claims 1 to 15 wherein said pressure sensor (20) is arranged into a sealed cavity (20') filled with a gas at a predetermined pressure.

17. The medical device (1) according to any one of claims 1 to 16 wherein the at least one pressure sensor (20) arranged to said outside surface (12b), comprises a protection layer that reduces considerably friction with body tissue and/or is biocompatible.

18. The medical device (1) according to any one of claims 1 to 17 wherein an electrical shielding is integrated to protect at least one sensing capacitor of said pressure sensor (20) and/or said electrical transmission means.
